(19) Europäisches Patentamt / European Patent Office / Office européen des brevets

(11) **EP 2 590 204 A1**

(12) **EUROPEAN PATENT APPLICATION**

(43) Date of publication:
**08.05.2013 Bulletin 2013/19**

(51) Int Cl.:
**H01J 37/26** (2006.01)      **G06F 17/30** (2006.01)
**G06F 17/40** (2006.01)

(21) Application number: **11187865.8**

(22) Date of filing: **04.11.2011**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(71) Applicant: **FEI Company
Hillsboro, OR 97124-5793 (US)**

(72) Inventors:
• **Faber, Pybe
5612 DV Eindhoven (NL)**
• **Groen, Bas
5612 HC Eindhoven (NL)**
• **Van Leeuwen, Hugo
5644 TH Eindhoven (NL)**
• **Van Niekerk, Koen
5615 AJ Eindhoven (NL)**
• **Verhoeve, Coert
5464 CN Veghel (NL)**

(74) Representative: **Bakker, Hendrik
FEI Company
Patent Department
P.O. Box 1745
5602 BS Eindhoven (NL)**

(54) **Charged-particle microscopy**

(57)      A method of investigating a sample using a charged-particle microscope, comprising the steps of:
- Providing a charged-particle microscope having a particle-optical column and a sample holder;
- Mounting the sample to the sample holder;
- Using the particle-optical column to direct an imaging beam of charged particles at the sample;
- Irradiating the sample with the imaging beam, as a result of which a flux of output radiation is caused to emanate from the sample;
- Examining at least a portion of said output radiation using a detector,

which method further comprises the following steps:
- Assigning sequential timestamps to detector data collected using said detector;
- For a given timestamp $T_n$, accumulating a set $\{M_n\}$ of examination measurands, each examination measurand $M_n$ representing a parameter value associated with a subsystem of the microscope and pertaining to timestamp $T_n$;
- For each timestamp $T_n$, saving the associated detector data $D_n$ together with the set $\{M_n\}$ of examination measurands to form a database C that has elements $E_n = (D_n, T_n, \{M_n\})$ and that is searchable on the basis of any search variable selected from the group $(T_n, \{M_n\})$.

Determine sequencing/spacing of timestamps $T_n$ to be used in timing "snapshots" of detector data $D_n$ and examination measurands $M_n$ at each $T_n$    S101

Choose constitution of set $\{M_n\}$ of examination measurands $M_n$, i.e. decide which measurands $M_n$ are to be "tracked" in each "snapshot"    S103

Provide sample on sample holder and irradiate it with imaging beam, thus producing output radiation that emanates from the sample    S105

Use a detector to examine at least a portion of the output radiation emanating from the sample, thus producing detector data. If desired, more than one detector can be deployed in this step    S107

At each timestamp $T_n$, accumulate values of the chosen examination measurands in the set $\{M_n\}$, and combine with the associated detector data $D_n$ to form a database element $E_n = (D_n, T_n, \{M_n\})$ that is stored in a database C    S109

**Fig. 2**

EP 2 590 204 A1

## Description

The invention relates to a method of investigating a sample using a charged-particle microscope, comprising the steps of:

- Providing a charged-particle microscope having a particle-optical column and a sample holder;
- Mounting the sample to the sample holder;
- Using the particle-optical column to direct an imaging beam of charged particles at the sample;
- Irradiating the sample with the imaging beam, as a result of which a flux of output radiation is caused to emanate from the sample;
- Examining at least a portion of said output radiation using a detector.

For purposes of clarity and consistency, the following terms as used throughout this text and the appended claims should be interpreted as follows:

- The term "charged particle" refers to an electron or ion (generally a positive ion, such as a Gallium ion or Helium ion, for example).
- The term "microscope" refers to an apparatus that is used to create a magnified image of an object, feature or component that is generally too small to be seen in satisfactory detail with the naked human eye. In addition to having an imaging functionality, such an apparatus may also have a machining functionality; for example, it may be used to locally modify a sample by removing material therefrom ("milling" or "ablation") or adding material thereto ("deposition"). Said imaging functionality and machining functionality may be provided by the same type of charged particle, or may be provided by different types of charged particle; for example, a Focused Ion Beam (FIB) microscope may employ a (focused) ion beam for machining purposes and an electron beam for imaging purposes (a so-called "dual beam" microscope, or "FIB-SEM"), or it may perform machining with a relatively high-energy ion beam and perform imaging with a relatively low-energy ion beam. On the basis of this interpretation, tools such as the following should be regarded as falling within the scope of the current invention: electron microscopes, FIB apparatus, EBID and IBID apparatus (EBID = Electron-Beam-Induced Deposition; IBID = ion-Beam-induced Deposition), etc.
- The term "particle-optical column" refers to a collection of electrostatic and/or magnetic lenses that can be used to manipulate a charged-particle beam, serving to provide it with a certain focus or deflection, for example, and/or to mitigate one or more aberrations therein.
- The term "sample holder" refers to any type of table, platform, arm, *etc.*, upon which a sample can be mounted and held in place. Generally, such a sample holder will be comprised in a stage assembly, with which it can be accurately positioned in several degrees of freedom, e.g. with the aid of electrical actuators.
- The term "output radiation" encompasses any radiation that emanates from the sample as a result of its irradiation by the imaging beam. Such output radiation may be particulate and/or photonic in nature. Examples include secondary electrons (SEs), backscattered electrons (BEs), X-rays, visible fluorescence light, and combinations of these. The output radiation may simply be a portion of the imaging beam that is transmitted through or reflected from the sample, or it may be produced by effects such as scattering or ionization, for example. In a special case, the phrase "output radiation" should be interpreted as comprising a flow ("flux") of electrical current (electrons) induced in the sample and flowing therefrom ("emanating") to a detector situated outside the sample, e.g. on the sample holder (as in the case of a so-called Electron Beam Induced Current (EBIC) set-up, for example).
- The term "detector" should be broadly interpreted as encompassing any detection set-up used to register (one or more types of) emitted radiation emanating from a sample. Such a detector may be unitary, or it may be compound in nature and comprise a plurality of sub-detectors, e.g. as in the case of a spatial distribution of detector units about a sample table, or a pixelated detector.

Such concepts will be familiar to the skilled artisan.

In what follows, the invention will - by way of example - often be set forth in the specific context of electron microscopes. However, such simplification is intended solely for clarity/illustrative purposes, and should not be interpreted as limiting.

Electron microscopy is a well-known technique for imaging microscopic objects. The basic genus of electron microscope has undergone evolution into a number of well-known apparatus species, such as the Transmission Electron Microscope (TEM), Scanning Electron Microscope (SEM), and Scanning Transmission Electron Microscope (STEM), and also into various sub-species, such as so-called "dual-beam" tools (e.g. a FIB-SEM), which additionally employ a "machining" beam of ions, allowing supportive activities such as ion-beam milling or ion-beam-induced deposition, for example. In traditional electron microscopes, the imaging beam is "on" for an extended period of time during a given imaging session; however, electron microscopes are also available in which imaging occurs on the basis of a relatively short "flash" or "burst" of electrons, such an approach being of potential benefit when attempting to image moving samples or radiation-sensitive specimens, for example.

In traditional use of an electron microscope, the microscope operator obtains, displays and interprets real-time imagery from a sample under investigation (whereby the term "imagery" should be broadly interpreted as including, for example, a spectrum, as well as the more traditional concept of an image/picture). Sometimes, if the operator sees something interesting in the investigation results, he may elect to save some of the obtained imagery on a data carrier, such as a hard disk or flash drive, for example; this, however, is discretionary, and in many cases the operator will not bother (or will simply forget) to do so. Even if he does save some imagery, re-locating it at a later stage, and remembering the exact context in which it was acquired, will often be problematic. This is particularly the case if saved imagery is recalled/interpreted by a different person to the person who originally saved it.

It is an object of the invention to address this issue. More specifically, it is an object of the invention to provide a method of operating a charged-particle microscope involving a revolutionary approach to data acquisition and management. More particularly, it is an object of the invention that such a method should increase the efficiency and versatility with which a charged-particle microscope can be used.

These and other objects are achieved in a method as specified in the opening paragraph, characterized by:

- Assigning sequential timestamps to detector data collected using said detector;
- For a given timestamp $T_n$, accumulating a set $\{M_n\}$ of examination measurands, each examination measurand $M_n$ representing a parameter value associated with a subsystem of the microscope and pertaining to timestamp $T_n$;
- For each timestamp $T_n$, saving the associated detector data $D_n$ together with the set $\{M_n\}$ of examination measurands to form a database C that has elements $E_n = (D_n, T_n, \{M_n\})$ and that is searchable on the basis of any search variable selected from the group $(T_n, \{M_n\})$.

In research leading to the invention, the inventors arrived at the insight that modern charged-particle microscopy (CPM) increasingly involves an element of accidental discovery, whereby a sample is observed to demonstrate a particular aspect or behavior that was not (fully) anticipated. Recent advances in CPM allow, for example, living biological cells to be observed, and such cells can demonstrate dynamic behavior at unpredictable times, such as cell division or necrosis, for example. Similarly, in the field of mineralogy, for example, chemical or physical reactions can occur at moments of time when the microscope operator was not expecting them. Alternatively, apart from such temporal aspects, a sample being investigated using CPM may unexpectedly undergo a change as a function of some other (instrumental/environmental) parameter in the microscope, such as beam energy, temperature, local atmospheric composition, *etc.*

Such dynamic behavior will often be irreversible, and the transitional process involved may be intrinsically fleeting, so that the microscope operator will often be caught off guard by a particular event. Moreover, the samples involved will often be expensive, rare or even unique. Consequently, the operator will generally not have the luxury of re-enacting the unexpected event in a fresh measurement. This leads to inefficiency, wasted time/resources, and a certain degree of observational blindness/helplessness as regards the samples/phenomena being investigated.

To address these problems, the inventors conceived a radical new way of processing measurement data from a CPM. Intrinsic to this invention are *(inter alia)* the following considerations:

(i) When something unexpected is observed, the operator should be able to "roll back time" as regards his measurement data. This will allow him to confirm the occurrence of an event (by comparing observational data to a previous/reference state), to (more) accurately identify the time of occurrence of the event, and/or to reveal a "time-lapse" of the actual occurrence of the event. Since the event in question is assumed to be unexpected, such a "roll back" in time will generally be most effective if microscope observations are *automatically* stored as a function of time, and are placed in a background database C for later reference; however, regular *manual* storage of measurement data in the database C is also conceivable.

(ii) Often, the operator will not be able to stipulate/estimate at what (exact) time a given event occurred, but he may well be able to (roughly) quantify another (instrumental/environmental) parameter associated with the occurrence of the event in question. For example, he may know/deduce that the event must have occurred at a particular temperature, gas pressure or beam current, based on his knowledge of the physical/chemical processes involved, or he may recall a transitional range of employed sample (holder) positions during which the event must have occurred, for example. In such cases, the database C referred to in point (i) above will be much more easily and effectively searchable if its elements $E_n$ contain not only a timestamp $T_n$ associated with particular detector data $D_n$, but also contain associated values of (one or more) examination measurands $M_n$ representing parameter values associated with (elected) subsystems of the microscope at timestamp $T_n$.

(iii) In addition to facilitating searching of the database C as a function of time, co-storing such measurands $M_n$ will allow purely non-temporal searching of the database C, allowing the operator to re-construct an evolutionary event in a sample as a function of some varying measurement parameter, e.g. concentration of a particular gaseous component being admitted to the CPM during measurement, or varying landing angle of the employed charged-particle beam, etc. (being just two specific examples from a whole scala of possibilities).

With reference to the terminology used in describing the current invention (e.g. as employed in the previous paragraph and in the Claims), the following should be explicitly noted:

- The chosen time interval $\Delta T$ between successive timestamps $T_n$ and $T_{n+1}$ is purely discretionary, and may be as small or as large as desired, or as dictated by the extent of the storage medium available for retention of the database C. One may also, if desired, work with variable values of AT. The basic concept of a "timestamp" should be familiar to the skilled artisan; however, if required, an elementary discourse on the subject can be found in the following Wikipedia article: http://en.vvikipedia.org/wiki/Timestamp

- A measurand $M_n$ may be a passive variable that is quantified by measurement, or an active variable that is quantified by setting. For example, in the case of $M_n$ = "sample holder position" (in a particular degree of freedom), it may be a *setpoint* for a positioning actuator of the holder (active variable), or it may be the *output* of a position measuring device, such as a linear encoder or interferometer (passive variable). A particular measurand $M_n$ may also be digital (binary) or analog in nature; an example of the former might be whether something is turned "on" or "off", or whether an instrument is "inserted" or "retracted", for example, whereas an example of the latter might be a (quasi-)continuous variable such as temperature or voltage, for example.

- When one refers to a (mathematical) "set" $\{M_n\}$ of measurands $M_n$, one should bear in mind that such a set can have just one member or plural members.

- In addition to the searchable measurands $M_n$ stored in the elements $E_n$ of the database C, one may also elect to co-store non-searchable measurands. Such measurands in this latter category might, for example, comprise screenshot data that are stored in a non-searchable bitmap form.

- Any of the variables $D_n$, $T_n$, $M_n$ may be stored in the database C as a "self-sufficient" value or as an incremental change to a reference value.

- The value of a variable $M_n$ and/or $D_n$ stored at timestamp $T_n$ need not be the value of that variable at the exact instant $T_n$, but may also be the most recent known value of that variable prior to $T_n$. For example, one can conceive one or more registers that store the most recent value(s) of the variable(s) in question, and that are continually updated as newer values of said variable(s) become available; in such a scenario, one could simply note and store the most up-to-date register value(s) of the indicated variables at timestamp $T_n$. Such a set-up might be used, for example, in the event of different clock rates being associated with some or all of the variables $T_n$, $M_n$, $D_n$.

- On a related note, the stored detector data $D_n$ at timestamp $T_n$ need not represent a momentary detector output at that timestamp, but may also represent a *cumulative* detector result up to that timestamp. For example, an image or spectrum presented on a display device will often be the result of a "scanning" or "accumulation" procedure, and will generally be built up of a data train that is produced and stored during a given time interval; in such an instance, $D_n$ will, in most practical situations, be the entire image/ spectrum that has been accumulated/stored up to timestamp $T_n$, but, if preferred, it may alternatively be a segment of said data train being produced or stored at timestamp $T_n$, or both. Similar considerations apply to a typical digital detector output, which, by its very nature, will generally be comprised of a data train of "1s" and "Os"; in such a scenario, the stored detector data at timestamp $T_n$ will typically be the last known full/composite digital value at $T_n$, rather than the momentary "1" or "0" in the underlying data train at the exact instant $T_n$; however, this does not have to be the case, and the current invention allows either scenario to be chosen, according to need or desire in a particular situation.

In a particular aspect of the last point discussed in the previous paragraph, detector data can undergo some sort of temporal processing, such as averaging, noise suppression, *etc.;* consequently, one can make a distinction between "manipulated detector data" and the underlying "raw" detector data from which they are obtained. According to a particular embodiment of the current invention, the detector data $D_n$ stored at timestamp $T_n$ are, in any case, the "raw" data -with the possible additional co-storage of the "manipulated" data, if desired. By storing the raw detector data in database C, one can perform whatever manipulations are desired or required at a later juncture.

As regards the set $\{M_n\}$ of examination measurands to be stored at each timestamp $T_n$ along with the associated detector data $D_n$, there are many ways in which the constitution of such a set $\{M_n\}$ (i.e. which measurands $M_n$ are to be included therein) could be decided. For example:

- The CPM might have a user interface (UI) that has a menu structure in a window or tab in which the operator can pre-select examination measurands $M_n$ whose value he wants to co-store with each timestamp $T_n$, e.g. by using an input device such as a mouse, trackball or touchpad to tick boxes in a list of candidate measurands $M_n$ presented on a computer display screen. The selected measurands are then "tracked" and their momentary values at each timestamp $T_n$ are stored as part of database entry

$E_n$ for that timestamp. If required, the operator can amend the members of the set $\{M_n\}$ as he wishes.

- Alternatively, one could conceive an automatic approach, in which the CPM itself determines which examination measurands to "track". One way to do this might be for the CPM to use interpretative software to elect measurands that it considers pertinent to a particular investigation session, and to ignore others. For example, the CPM might be pre-programmed to know that there's no point in tracking ion beam current during a purely electron beam investigation, or that there's no point in tracking parameters of a SE detector if the operator is only performing X-ray detection during a given session.
- As yet another alternative, one could envisage a blind "save everything" approach, in which all available measurands are stored in database C at each timestamp $T_n$.

The skilled artisan will be well able to implement such scenarios, and to conceive others, such as various hybrids of these approaches.

Once the constitution of the set $\{M_n\}$ of examination measurands to be "tracked" has been established, one could conceive a UI in which the operator simply clicks on (or presses) a "save" button at regular intervals, whereupon the CPM saves an entry $E_n = (D_n, T_n, \{M_n\})$ to the database C, including the values of $D_n$ and $\{M_n\}$ at the timestamp $T_n$ at which the "save" operation was enacted; such an approach involves a *manual* trigger to instigate the "save" operation (although the actual data $\{M_n\}$ stored in each element $E_n$ are determined/collected according to a preceding selection step, and are thus a non-manual part of the "save" operation). However, in an alternative embodiment of the method according to the current invention, the elements $E_n$ are *automatically* saved in said database for a sequence of consecutive timestamps $T_n$ spanning a given period of time T. Such a possibility has already been alluded to under point (i) above, and has *interalia* the following advantages:

- Automated compilation of the database C is not affected by "human-error issues" such as forgetfulness, fatigue, lack of discipline, *etc.,* on the part of the operator; the database is "invisibly" filled in a background procedure, allowing the operator to concentrate on other aspects of his work.
- Non-manual compilation generally allows much more frequent timestamps $T_n$ to be chosen than in the case of manual "save" operations, and even allows for *continuous* saving of data.

In such a scenario, one could again conceive a UI menu structure in which an operator can pre-select a value of the time interval AT to be observed between successive timestamps $T_n$ and $T_{n+1}$. Alternatively and/or in supplement hereto, the CPM might comprise software/firmware that can autonomically decide to take a "snapshot" $E_n = (D_n, T_n, \{M_n\})$ at a timestamp $T_n$ corresponding to (the commencement of) some significant transitional event in a sample investigation session, such as an adjustment to an imaging beam parameter, sample table position, *etc.* In both these scenarios, one could envisage a (quasi-)continuous "time-lapse" recording of database elements $E_n$ during some significant event (such as cell division, for example), whether such a recording is operator-instigated or autonomous (such a time-lapse recording basically being a special time interval during which $\Delta T$ is (temporarily) chosen to be very small).

In recent years, the price of electronic memories (magnetic, optical, magnetooptical, semiconductor, *etc.)* has reduced greatly, allowing storage devices with very large storage capacity (e.g. several terabytes) to be acquired at relatively low cost; consequently, without the need to invest in very expensive storage capacity, a database C as set forth in the context of the current invention can be allowed to achieve relatively large proportions. However, sooner or later, the database C will (threaten to) become "full" in the sense that it will no longer fit on the available data carrier used to store it. In that case, one may elect to flush (or "prune") the database C on the basis of a "First In, First Out" (FIFO) approach. Accordingly, in an embodiment of the current invention, the database C is automatically flushed of older elements $E_n$ on the basis of an algorithm selected from the group comprising:

- Removing elements $E_n$ having a timestamp $T_n$ that precedes a chosen reference timestamp $T_{ref}$ by more than a particular span of time; or
- If the total volume S of data in the database C exceeds a chosen reference size $S_{ref}$, removing one or more elements $E_n$ so as to restore the condition $S < S_{ref}$, starting with those elements $E_n$ having the oldest timestamp $T_n$,

and combinations hereof.

It should be realised that data flushed out of database C in this manner need not be discarded altogether; instead, if the operator/owner of the CPM so wishes, flushed data can be archived on "static" storage media (e.g. DVDs or magnetic reels), allowing more "dynamic" storage media (e.g. hard disk drives or flash memory) to be used to store the more recent (i.e. un-flushed) portions of database C. It should also be realised that a flushing operation as set forth here may be fully automatic, operator-instigated, operator-performed, or combinations hereof, according to choice. The skilled artisan will appreciate this point, and will be able to embody such a flushing procedure according to the needs of a particular situation. He will also realize that there are various other candidate algorithms that can be used to flush and/or compact the database C.

The dissertation above has already set forth various measurands $M_n$ that can be "tracked", timestamped and stored in the context of the current invention.

In particular, one or more of the following measurands may prove to be of especial importance when investigating a sample:

- Position of the sample holder.
  The sample holder will generally be positionable in multiple Degrees of Freedom (DOFs), e.g. any or all of linear DOFs $X$, $Y$ and $Z$ (in a Cartesian co-ordinate system) and associated angular DOFs $Rx$, $Ry$ and $Rz$ (roll, pitch and yaw). Noting the (multi-DOF) position of the sample holder as an examination measurand $M_n$ will help in determining where on a (static) sample a particular signal of interest was captured. It will, for example, also enable the landing angle of the imaging beam on the sample to be determined.
- Energy of the imaging beam.
  Varying the (landing) energy of the imaging beam will *inter alia* influence the depth to which the imaging beam will penetrate into the sample. Beam energy can also play a role in influencing whether certain chemical/physical reactions are triggered or not, since such reactions may require an input of energy above a certain threshold. Noting the beam energy as an examination measurand $M_n$ can thus help determine from what subsurface depth within a sample a particular signal was captured, or what the endoergic threshold is of a particular observed reaction.
- Electrical current in the imaging beam.
  This determines the intensity (energy density) of the imaging beam, which in turn plays an important role *inter alia* in radiation damage processes, particularly in biological samples; tracking the beam current as an examination measurand $M_n$ will facilitate the investigation of such processes. In addition, the magnitude of the beam current plays a role in sample charging effects, which in turn influence image contrast. Moreover, adjusting the beam current tends to have opposite effects on Signal-to-Noise ratio (SNR) and image resolution. Being able to track beam current as an examination measurand $M_n$ therefore yields valuable information on the state of the sample and various general imaging parameters.
- Type of charged particle in the imaging beam.
  Electrons and (different types of) ions have radically different effects on samples. For example, ions (which are thousands of times more massive than electrons) tend to cause more radiation damage in the sample, tend to ablate (more) material therefrom, and can cause "swelling" when they become implanted in the crystalline lattice of a sample. Electron and ion beams also tend to produce radically different image contrast. Knowing the type of charged particle used (or not used) to acquire imagery, and/or knowing at what point a switch was made from one type of charged particle to another, can greatly help to re-locate and/or interpret particular imagery results.
- Field of view of the particle-optical column.

This measurand can also be referred to as the scan field width (where scanning is relevant), or magnification, for example. Tracking its value as an examination measurand $M_n$ can help determine the location on a sample from which a particular signal was measured. Additionally, it can help ensure that reference imagery retrieved from database C will have a "zoom ratio" that is the same as, or similar to, that of query imagery to which the reference imagery is to be compared.

- Temperature (in the vicinity) of the sample.
  As in the case of beam energy, temperature of the sample will generally play a role in the occurrence of certain chemical/physical reactions, and/or in the occurrence of certain biological processes, such as cell division. Being able to recall the temperature (whether measured or inferred, either at the sample or in its vicinity) as an examination measurand $M_n$ will often play an important role in the interpretation of the associated detector data $D_n$ in database C.
- Partial gas pressure at the location of the sample.
  Many CPMs strive to maintain as good a vacuum as possible in the vicinity of a sample under investigation, but other types of CPM (such as so-called atmospheric or environmental SEMs, for example) deliberately administer gas(es) to the sample vicinity. Being able to recall:

  (1) Whether or not there was any gas administered;
  (2) If so, which gas(es); and, if possible,
  (3) The (partial) pressure(s) of the gas(es) concerned,

  will generally provide valuable information in interpreting detector data $D_n$ retrieved from the database C. Any or all of items (1)-(3) can be tracked as examination measurands $M_n$ in the context of the current invention.

- Spatial configuration of the detector with respect to the sample.
  Some CPMs employ multiple detectors of a given type in an attempt to spatially resolve output radiation emanating from a sample; this can be referred to as a "cloud configuration" of detectors. In interpreting detector data $D_n$ pertaining to a given investigation session, it can be useful to know from which detector in such a "cloud configuration" particular data were obtained. Alternatively, even in the case of a single detector of a given type, it can be useful to know where that detector was located with respect to the sample being investigated.

This list is given by way of example only, and should not be seen as being exhaustive. Indeed, the skilled artisan will appreciate that there are many other possible measurands $M_n$ that can be listed in the current context and that fall within the scope of the current invention and claims. For example:

- The applied force in a tensile (sub-)stage used to position the sample holder.
- Whether a sample examination probe and/or gas administration needle is inserted or retracted.
- The value of an electrical voltage applied to the sample.
- The flow velocity of liquid in a flow-type sample holder (e.g. of a type as marketed by the firm *Hummingbird* Scientific in Washington State, USA (www.hummingbirdscientific.com)).
- A "mode" of the particle-optical column, e.g. low or high current mode, low or high resolution mode, monochromator on or off, aberration correction on or off, *etc.,*

to name but a few possibilities.

The detector yielding the detector data $D_n$ that is stored in database C can be any type of detector employable in a CPM. Examples include a secondary electron (SE) detector, a backscattered electron (BE) detector, a secondary ion detector, an X-ray detector, an EDS (Energy Dispersive Spectroscopy) detector (which may be regarded as being a type of X-ray detector), a fluorescence detector, an SPM (Scanning Probe Microscopy) detector, an AFM (Atomic Force Microscopy) detector (which may be regarded as being a type of SPM), a Raman spectroscope, an Auger spectroscope, and an ammeter (as in the case of EBIC, for example). Within a particular CPM, a given detector from this group may occur singularly or plurally (e.g. as in the case of the "cloud configuration" referred to above). Generally, a CPM will contain several different types of detector, to allow different types/aspects of output radiation emanating from the sample to be investigated, e.g. it may contain detectors for examining SEs, BEs, X-rays and fluorescence light.

In a particular embodiment of a method according to the current invention, a given element $E_n$ of the database C contains detector data $D_n$ obtained from more than one detector. For example, it would be very useful if, while an operator were *actively* investigating a sample using a BE detector (accumulating detector data of type $D_n^1$), detector data from an SE detector in the same CPM were being concurrently *passively* stored in the background (accumulating detector data of type $D_n^2$); after all, both detectors are "on", and, although the operator may only have the resources to actively concentrate on the output from one detector, there is often no reason why the data from another detector cannot be passively accumulated and stored for possible later perusal (together with the associated timestamps $T_n$ and examination measurands $M_n$). Such a scenario would greatly increase the efficiency and versatility with which a CPM can be employed.

In a particular aspect of the scenario set forth in the previous paragraph, the following applies:

- Output radiation emanating from the sample is examined using at least first and second detectors, $O_1$ and $O_2$;
- These detectors yield respective *raw* detector data $D_n^1$ and $D_n^2$ at timestamp $T_n$;
- These raw detector data $D_n^1$ and $D_n^2$ are processed to produce *manipulated* detector data $D_n'$ for timestamp $T_n$;
- At least the *raw* detector data $D_n^1$ and $D_n^2$ are stored in element $E_n$ of the database C for timestamp $T_n$;
- Optionally, the manipulated detector data $D_n'$ are also stored in element $E_n$ for timestamp $T_n$.

This aspect of the invention will be explained on the basis of the following specific example, in which there are four detectors, $O_1$, $O_2$, $O_3$ and $O_4$, e.g. quadrant detectors for electron detection. With such a set-up, one can conceive many different manipulation possibilities as regards the raw detector data $D_n^1$, $D_n^2$, $D_n^3$ and $D_n^4$ output from these detectors. For example, one might be interested in a total sum signal:

$$^S D_n' = D_n^1 + D_n^2 + D_n^3 + D_n^4$$

or one might be more interested in a compound difference signal, such as:

$$^D D_n' = (D_n^1 + D_n^2) - (D_n^3 + D_n^4)$$

Since there are several detectors, there are many different combination possibilities for the raw detector data $D_n^1$, $D_n^2$, $D_n^3$ and $D_n^4$. By storing the raw detector data in database C, any one of these (manipulated) combinations can be constructed and examined at a later stage, at will.

In another possible example involving a "cloud configuration" of detectors as referred to above, a conceivable manipulation of detector data might involve (weighted) spatial averaging of the raw detector data, whereby one attaches less importance to peripheral detectors and attaches more importance to paraxial detectors, for example. By storing the raw detector data in database C, one can perform whatever manipulations are desired or required at a later juncture.

In the context of this discussion, it should be noted that the first and second detectors, $O_1$ and $O_2$ referred to here may, in fact, be different regions (*e.g.* segments) of a single detection surface.

When there is a need to consult a database C compiled in accordance with the present invention, one could, for example, envisage a UI feature similar to that already set forth above in respect of choosing the elements of the set $\{M_n\}$. In such a scenario, the UI has a menu structure (e.g. in a window or tab) in which the operator can:

- Pre-select a range of timestamp values $T_n$ to which a search is to be confined; and/or
- Pre-select examination measurands $M_n$ to be used in searching the database C (e.g. by ticking boxes in a list of candidate measurands $M_n$ presented on a computer display screen). If desired, the operator can also stipulate a value range to be used for each given measurand $M_n$ in the search in question.

According to the invention, where there is a desire to search the database C for data relating to a particular observation, an operator may, for example, be able to do one or more of the following:

- *Remember* a particular (approximate) value range of a given measurand $M_n$ and/or timestamp $T_n$ associated with the observation in question;
- *Infer* a particular value range of $M_n$ and/or $T_n$, based on understanding of a mechanism underlying said observation;
- *Eliminate* a particular value range of $M_n$ and/or $T_n$, based on logical exclusion, any or all of which actions can be used to narrow/expedite the search for the observation in question.

In principle, the database C could also be searched on the basis of search ranges pertaining to *detector* data $D_n$, rather than just variables $T_n/M_n$. For example, one could search the database C for the occurrence of a particular spectral line, or a particular line width (e.g. FWHM), or on the basis of a particular threshold intensity, *etc.*

Apart from performing a pure numeric search of the database C compiled according to the invention, one could also make good use of its contents in other manners. For example, one could invoke a plot/graph of stored detector data $D_n$ as a function of timestamp $T_n$ or a given measurand $M_n$. Such a graphical representation will often more vividly reveal particular tendencies within accumulated data.

As a further possibility, one could envisage a scenario whereby a computer processor could automatically indicate to an operator those measurands (stored in the database C) which underwent a (significant) change in value during a particular timespan or event. This would delegate some of the burden of data analysis from the operator to the processor/CPM, which would thus be given a more proactive role in perusing/interpreting the database C.

The invention will now be elucidated in more detail on the basis of exemplary embodiments and the accompanying schematic drawings, in which:

Figure 1 renders a longitudinal cross-sectional view of part of a (particular type of) charged-particle microscope in which a method according to the present invention can be enacted;

Figure 2 renders a flowchart depiction of a particular embodiment of a method according to the current invention.

In the Figures, where relevant, corresponding parts are indicated using corresponding reference symbols.

Embodiment 1

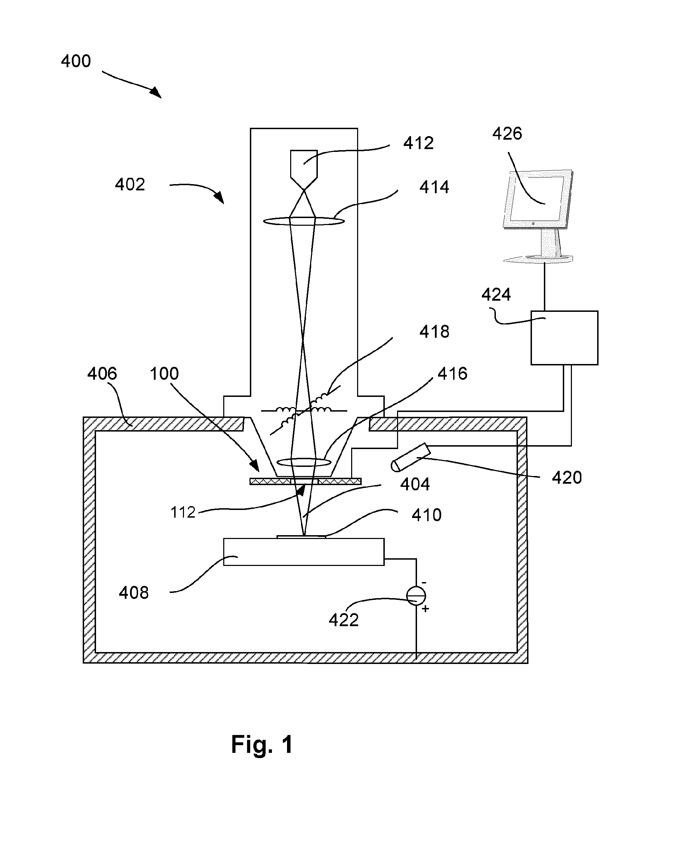

Figure 1 shows a charged-particle microscope (CPM) 400 in which a method according to the current invention can be implemented. In this case, the depicted CPM is a SEM. The microscope 400 comprises a particle-optical column 402, which produces an imaging beam 404 of charged particles (in this case, an electron beam). The particle-optical column 402 is mounted on a vacuum chamber 406, which comprises a sample holder / stage 408 for holding a sample 410. The vacuum chamber 406 is evacuated using vacuum pumps (not depicted). With the aid of voltage source 422, the sample holder 408, or at least the sample 410, may be biased (floated) to an electrical potential with respect to ground.

The particle-optical column 402 comprises an electron source 412, lenses 414, 416 to focus the imaging beam 404 onto the sample 410, and a deflection unit 418. As regards detectors, the apparatus is equipped with:

- A first detector 420, for detecting a first type of stimulated emitted radiation emanating from the sample 410 in response to irradiation by the imaging beam 404. In the present example, the detector 420 is an X-ray detector (such as an EDS or WDS detector) for detecting X-rays.
- A second detector 100, for detecting a second type of stimulated radiation emitted from the sample 410 in response to irradiation by the imaging beam 404. In the present example, the detector 100 is a segmented electron detector.

As here depicted, the apparatus uses both of these detector types; however, this is purely a design/implementation choice and, if desired, it's also possible to use just one of these detectors types. The apparatus further comprises a computer processing apparatus (controller) 424 for controlling *inter alia* the deflection unit 418, lenses 414, and detectors 420,100, and displaying information gathered from the detectors 420,100 on a display unit 426.

By scanning the imaging beam 404 over the sample 410, stimulated radiation - comprising, for example, X-rays, secondary electrons (SEs) and backscattered electrons (BEs) - emanates from the sample 410. X-rays are detected by first detector 420, whereas SEs/BEs are detected by second detector 100. As the emitted radiation is position-sensitive (due to said scanning motion), the information obtained from the detectors 420, 100, will also be position-dependent.

The signals from the detectors 420,100 are processed by the processing apparatus 424, and displayed on display unit 426. Such processing may include operations such as combining, integrating, subtracting, false colouring, edge enhancing, and other processing known to the person skilled in the art. In addition, automated recognition processes, e.g. as used for particle analysis, may be included in such processing.

Many refinements and alternatives of such a set-up will be known to the skilled artisan, including, but not limited to, the detection of (infrared/visible/ultraviolet) light emanating from the sample 410, the use of dual beams (for example an electron beam 404 for imaging and an ion beam for machining (or, in some cases, imaging) the sample 410), the use of a controlled environment at the sample 410 (for example, maintaining a pressure of several mbar- as used in a so-called Environmental SEM - or by admitting gasses, such as etching or precursor gasses), etc.

In a CPM such as this, a method according to the current invention can be implemented, as will now be set forth in more detail.

Embodiment 2

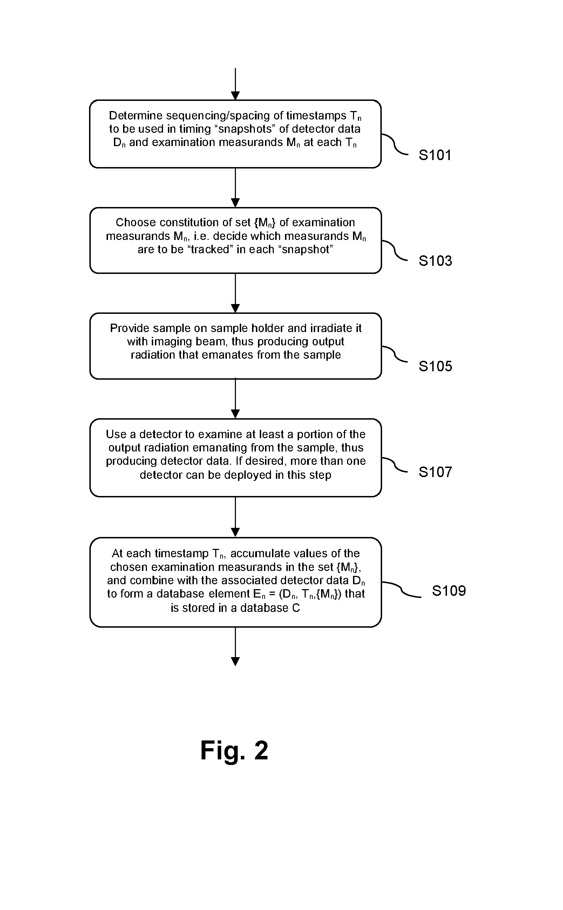

Figure 2 depicts a flowchart of a particular embodiment of a method according to the current invention. The various steps S101-S109 in this flowchart can be elucidated as follows.

- *S101:* In this step, one basically decides the frequency (and phase) of timestamps $T_n$ to be used as a timing "grid" - either for application during a particular sample investigation session, or to be stored as a "machine constant" for default application in all investigations performed with a given CPM. As set forth above, this can be done using, for example, a UI menu to pre-select a value of the time interval $\Delta T$ to be observed between successive timestamps $T_n$ and $T_{n+1}$. Alternatively and/or in supplement hereto, the CPM might be provided with (input to) software/ firmware that can autonomically decide to (continually) appoint a timestamp $T_n$ to (the commencement of) some significant transitional event in a sample investigation session, such as an adjustment to an imaging beam parameter, sample table position, etc. The (initially) planned grid of timestamps thus decided may be "fluidic" (i.e. dynamic) or "rigid" (i.e. static). In the former case, (automatic or manual) adjustment can be made to the grid "on the fly", in order to accommodate (unexpected) changes as a sample investigation evolves: one could, for example, envisage a (quasi-)continuous "time-lapse" recording of detector data $D_n$ during some significant event, such a time-lapse recording involving a $\Delta T$-value that is (temporarily) chosen to be very small.

- *S103:* In this step, one chooses what examination measurands are to be tracked and recorded during the "snapshots" referred to in the dissertation above, i.e. one determines the constitution (make-up) of the set $\{M_n\}$. As already set forth above in more detail, this choice can be performed manually or (quasi-) automatically. Just as in step S101, the chosen constitution of $\{M_n\}$ can be specific (to a particular investigation session) or general (to a given CPM tool), and can be dynamic or static.

- *S105:* In this step, the actual investigation of a sample commences: the sample is mounted on a sample holder within the CPM, positioned as desired, and irradiated with an imaging beam of charged particles, thus producing output radiation that emanates from the sample.

- *S107:* In this step, (at least one type of) output radiation as referred to in step S105 is examined using at least one detector. Regardless of whether the detector in question is used to form an image or a spectrum of the sample, for example, it can in any case be said to produce detector data (which may be digital or analog, for example).

- *S109:* In this step, the timing grid set-up in step S101 is used as a reference to take data "snapshots" that are stored in a database C. In such a "snapshot" at timestamp $T_n$, the (momentary) values of the detector data $D_n$ and of each of the measurands $M_n$ in the set $\{M_n\}$ are compiled together with $T_n$ itself into a database element (entry) $E_n = (D_n, T_n, \{M_n\})$.

Automated / computer-controlled aspects of the steps S101-S109 can be performed with the assistance of, for example, a computer processing apparatus (controller) 424 as referred to above in Embodiment 1.

In accordance with the present invention, the database C compiled in this manner is searchable on the basis of timestamp and one or more measurands in the set $\{M_n\}$. Once again, such a search can, for example, be conducted using a UI menu feature in which one or more search variables are selected, possibly together with target value ranges for the selected search variables. For example, one might search the database C for detector data accumulated:

- In the timestamp range: June 24, 2011, 14:00 - 17:00 GMT; and
- In the beam energy range: 4.5-6.2 keV; and
- For a sample temperature above 34°C.

**Claims**

1. A method of investigating a sample using a charged-particle microscope, comprising the steps of:

   - Providing a charged-particle microscope having a particle-optical column and a sample holder;
- Mounting the sample to the sample holder;
- Using the particle-optical column to direct an imaging beam of charged particles at the sample;
- Irradiating the sample with the imaging beam, as a result of which a flux of output radiation is caused to emanate from the sample;
- Examining at least a portion of said output radiation using a detector,

**characterized by**:

- Assigning sequential timestamps to detector data collected using said detector;
- For a given timestamp $T_n$, accumulating a set $\{M_n\}$ of examination measurands, each examination measurand $M_n$ representing a parameter value associated with a subsystem of the microscope and pertaining to timestamp $T_n$;
- For each timestamp $T_n$, saving the associated detector data $D_n$ together with the set $\{M_n\}$ of examination measurands to form a database C that has elements $E_n = (D_n, T_n, \{M_n\})$ and that is searchable on the basis of any search variable selected from the group $(T_n, \{M_n\})$.

2. A method according to claim 1, wherein the elements $E_n$ are automatically saved in said database for a sequence of consecutive timestamps $T_n$ spanning a given period of time T.

3. A method according to claim 1 or 2, wherein said database C is automatically flushed of older elements $E_n$ on the basis of an algorithm selected from the group comprising:

- Removing elements $E_n$ having a timestamp $T_n$ that precedes a chosen reference timestamp $T_{ref}$ by more than a particular span of time; or
- If the total volume S of data in the database C exceeds a chosen reference size $S_{ref}$, removing one or more elements $E_n$ so as to restore the condition $S < S_{ref}$, starting with those elements $E_n$ having the oldest timestamp $T_n$, and combinations hereof.

4. A method according to any preceding claim, wherein said set $\{M_n\}$ of examination measurands has at least one element selected from the group comprising position of the sample holder, energy of the imaging beam, electrical current in the imaging beam, type of charged particle in the imaging beam, field of view of the particle-optical column, temperature of the sample, partial gas pressure at the location of the sample, spatial configuration of the detector with respect to the sample, and combinations hereof.

5. A method according to any preceding claim, wherein said detector is selected from the group comprising a secondary electron detector, a backscattered electron detector, a secondary ion detector, an X-ray detector, an EDS detector, a fluorescence detector, an SPM detector, an AFM detector, a Raman spectroscope, an Auger spectroscope, an ammeter, and combinations hereof.

6. A method according to any preceding claim, wherein a given element $E_n$ of the database C contains detector data $D_n$ obtained from more than one detector.

7. A method according to claim 6, wherein:

- Output radiation emanating from the sample is examined using at least first and second detectors;
- These detectors yield respective raw detector data $D_n^1$ and $D_n^2$ at timestamp $T_n$;
- These raw detector data $D_n^1$ and $D_n^2$ are processed to produce manipulated detector data $D_n'$ for timestamp $T_n$;
- At least the raw detector data $D_n^1$ and $D_n^2$ are stored in element $E_n$ of the database C for timestamp $T_n$.

8. A method according to any of the claims 1-5, wherein:

- Raw detector data $D_n$ are accumulated during a timespan prior to timestamp $T_n$;
- These raw detector data undergo temporal processing to produce manipulated detector data $D_n'$;
- At least the raw detector data $D_n$ are stored in element $E_n$ of the database C for timestamp $T_n$.

9. A charged-particle microscope constructed and arranged to perform a method as claimed in any of the claims 1-8.

**Fig. 1**

Determine sequencing/spacing of timestamps $T_n$
to be used in timing "snapshots" of detector data
$D_n$ and examination measurands $M_n$ at each $T_n$

S101

Choose constitution of set $\{M_n\}$ of examination
measurands $M_n$, i.e. decide which measurands $M_n$
are to be "tracked" in each "snapshot"

S103

Provide sample on sample holder and irradiate it
with imaging beam, thus producing output
radiation that emanates from the sample

S105

Use a detector to examine at least a portion of the
output radiation emanating from the sample, thus
producing detector data. If desired, more than one
detector can be deployed in this step

S107

At each timestamp $T_n$, accumulate values of the
chosen examination measurands in the set $\{M_n\}$,
and combine with the associated detector data $D_n$
to form a database element $E_n = (D_n, T_n, \{M_n\})$ that
is stored in a database C

S109

# Fig. 2

Europäisches
Patentamt

European
Patent Office

Office européen
des brevets

**EUROPEAN SEARCH REPORT**

Application Number

EP 11 18 7865

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2007/274609 A1 (HIRAI TAKEHIRO [JP] ET AL) 29 November 2007 (2007-11-29) * paragraphs [0005], [0011], [0012], [0017], [0027], [0028], [0037], [0039] - [0042], [0044]; figures 1,4-6 * | 1,5,9 | INV. H01J37/26 G06F17/30 G06F17/40 |
| X | US 2009/242765 A1 (MAEDA TATSUYA [JP]) 1 October 2009 (2009-10-01) * paragraphs [0029], [0030], [0056], [0060] - [0066], [0075] - [0083], [0091] - [0095], [0101] - [0104]; figures 2,4,5,7,8 * | 1-5,9 | |
| X | US 2003/193025 A1 (TAKAGI SHIGENORI [JP]) 16 October 2003 (2003-10-16) * paragraphs [0117] - [0123], [0192] - [0193], [0209] - [0210], [0253], [0279]; figures 1,31 * | 1-6,9 | |
| A | US 2007/023658 A1 (NOZOE MARI [JP] ET AL) 1 February 2007 (2007-02-01) * paragraph [0054]; figure 1 * | 5 | TECHNICAL FIELDS SEARCHED (IPC) H01J G06F |
| A | US 2011/001816 A1 (YONEKURA ISAO [JP] ET AL) 6 January 2011 (2011-01-06) * paragraphs [0008] - [0009] * | 5 | |

~~The present search report has been drawn up for all claims~~

| Place of search | Date of completion of the search | Examiner |
|---|---|---|
| Munich | 5 April 2012 | Krauss, Jan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

Application Number

EP 11 18 7865

---

**CLAIMS INCURRING FEES**

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

**LACK OF UNITY OF INVENTION**

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-3, 5, 6, 9(completely); 4(partially)

☐ The present supplementary European search report has been drawn up for those parts of the European patent application which relate to the invention first mentioned in the claims (Rule 164 (1) EPC).

Europäisches
Patentamt
European
Patent Office
Office européen
des brevets

**LACK OF UNITY OF INVENTION
SHEET B**

**Application Number**

EP 11 18 7865

The Search Division considers that the present European patent application does not comply with the requirements of unity of invention and relates to several inventions or groups of inventions, namely:

1. claims: 1-3, 5, 6, 9(completely); 4(partially)

   dedicated to a method of investigating a sample wherein the elements E n are automatically saved as defined in claim 2
   ---

2. claim: 4(partially)

   dedicated to a method of investigating a sample with the alternative of claim 4 that the set of examination measurands has at least one element that is the temperature of the sample
   ---

3. claims: 7, 8

   dedicated to a method of investigating a sample wherein raw data is stored as defieined in present claims 7 or 8
   ---

**ANNEX TO THE EUROPEAN SEARCH REPORT
ON EUROPEAN PATENT APPLICATION NO.**

EP 11 18 7865

This annex lists the patent family members relating to the patent documents cited in the above-mentioned European search report.
The members are as contained in the European Patent Office EDP file on
The European Patent Office is in no way liable for these particulars which are merely given for the purpose of information.

05-04-2012

| Patent document cited in search report | | Publication date | Patent family member(s) | | Publication date |
|---|---|---|---|---|---|
| US 2007274609 | A1 | 29-11-2007 | NONE | | |
| US 2009242765 | A1 | 01-10-2009 | JP | 4579712 B2 | 10-11-2010 |
| | | | JP | 2006236700 A | 07-09-2006 |
| | | | US | 2006188216 A1 | 24-08-2006 |
| | | | US | 2009242765 A1 | 01-10-2009 |
| US 2003193025 | A1 | 16-10-2003 | JP | 4014917 B2 | 28-11-2007 |
| | | | JP | 2003303567 A | 24-10-2003 |
| | | | US | 2003193025 A1 | 16-10-2003 |
| US 2007023658 | A1 | 01-02-2007 | JP | 4312910 B2 | 12-08-2009 |
| | | | JP | 2001159616 A | 12-06-2001 |
| | | | US | 2001017878 A1 | 30-08-2001 |
| | | | US | 2003213909 A1 | 20-11-2003 |
| | | | US | 2005006583 A1 | 13-01-2005 |
| | | | US | 2007023658 A1 | 01-02-2007 |
| US 2011001816 | A1 | 06-01-2011 | CN | 101978241 A | 16-02-2011 |
| | | | KR | 20100126398 A | 01-12-2010 |
| | | | US | 2011001816 A1 | 06-01-2011 |
| | | | WO | 2009116634 A1 | 24-09-2009 |

EPO FORM P0459

For more details about this annex : see Official Journal of the European Patent Office, No. 12/82